(19) Europäisches Patentamt
European Patent Office
Office européen des brevets

(11) EP 1 803 465 A1

(12) **EUROPEAN PATENT APPLICATION**
published in accordance with Art. 158(3) EPC

(43) Date of publication:
**04.07.2007 Bulletin 2007/27**

(21) Application number: **05783950.8**

(22) Date of filing: **06.09.2005**

(51) Int Cl.:
***A61K 38/46*** *(2006.01)* ***A61P 1/16*** *(2006.01)*

(86) International application number:
**PCT/CN2005/001411**

(87) International publication number:
**WO 2006/026915 (16.03.2006 Gazette 2006/11)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.09.2004 CN 200410076854**

(71) Applicant: **Bio-Cancer Treatment International Limited**
**Hong Kong (CN)**

(72) Inventor: **CHENG, Ning Man**
**Hong Kong (CN)**

(74) Representative: **Goodfellow, Hugh Robin et al**
**Carpmaels & Ransford**
**43-45 Bloomsbury Square**
**London WC1A 2RA (GB)**

(54) **PHARMACEUTICAL COMPOSITION AND METHOD OF TREATING HEPATITIS WITH ARGINASES**

(57) The invention discloses methods for treating hepatitis with human arginase I modified by polyethylene glycol and uses of it in manufacturing of a medicament.

```
   1 gaattgtacg tcaaagagat gaagcagaaa aacgtcgtcg agaagaagct gaacgacaaa
  61 aagtgaaatg cgagggaagt ccaagaaatg gtgattatga gggtgtctat ttcaccaaaa
 121 acggagaata tttattggaa ttaagagtct ctgggactgc tcttgtaaat gctccttgta
 181 atttaaagga tattgacata acgaaatggt tgtgtaaaac agggagatta tatcttgata
 241 aggttaagaa atttgaaata gttactattc tttcccatga cgtagaaaat caaaagatta
 301 taacagaatg ggagtcactc cccagagagg ctttacccga acaatttgat tcataagaac
 361 taattagtag cgctttccaa tggaggcgct tttttatttg ggtagttgca taccactaaa
 421 gatgttcagg tgcacatgag cattggagga aaggaacgct ttagggggaa gggaaacctt
 481 taaacagtct taatccccct tgattttatg ttctctgtaa actgcgtccg gtaaatctca
 541 ggatagacaa tcggcggtta acggcttgag tgcgggggca gtttagaaag aatatgattg
 601 gagggattca tagatgcatc accatcacca tcatatgagc gccaagtcca gaaccatagg
 661 gattattgga gctcctttct caaagggaca gccacgagga ggggtggaag aaggccctac
 721 agtattgaga aaggctggtc tgcttgagaa acttaaagaa caagagtgtg atgtgaagga
 781 ttatggggac ctgccctttg ctgacatccc taatgacagt ccctttcaaa ttgtgaagaa
 841 tccaaggtct gtgggaaaag caagcgagca gctggctggc aaggtggcac aagtcaagaa
 901 gaacggaaga atcagcctgg tgctgggcgg agaccacagt ttggcaattg gaagcatctc
 961 tggccatgcc agggtccacc ctgatcttgg agtcatctgg gtggatgctc acactgatat
1021 caacactcca ctgacaacca caagtggaaa cttgcatgga caacctgtat ctttcctcct
1081 gaaggaacta aaaggaaaga ttcccgatgt gccaggattc tcctgggtga ctccctgtat
1141 atctgccaag gatattgtgt atattggctt gagagacgtg gaccctgggg aacactacat
1201 tttgaaaact ctaggcatta aatacttttc aatgactgaa gtggacagac taggaattgg
1261 caaggtgatg gaagaaacac tcagctatct actaggaaga aagaaaaggc caattcatct
1321 aagttttgat gttgacggac tggacccatc tttcacacca gctactggca caccagtcgt
1381 gggaggtctg acatacagag aaggtctcta catcacagaa gaaatctaca aaacagggct
1441 actctcagga ttagatataa tggaagtgaa cccatccctg ggaagacac cagaagaagt
1501 aactcgaaca gtgaacacag cagttgcaat aaccttggct tgtttcggac ttgctcggga
1561 gggtaatcac aagcctattg actaccttaa cccacctaag taaatgtgga aacatccgat
1621 ataaatctca tagttaatgg cataattaga aagctaatca ttttcttaag catagagtta
1681 tccttctaaa gacttgttct ttcagaaaaa tgtttttcca attagtataa actctacaaa
1741 ttccctcttg gtgtaaaatt caagatgtgg aaattctaac tttttgaaa tttaaaagct
1801 tatattttct aacttggcaa aagacttatc cttagaaaga gaagtgtaca ttgatttcca
1861 attaaaaatt tgctggcatt aaaaataagc acacttacat aagcccccat acatagagtg
1921 ggactcttgg aatcaggaga caaagctacc acatgtggaa aggtactatg tgtccatgtc
1981 attcaaaaaa tgtgattcta ga
```

Fig. 1A



Printed by Jouve, 75001 PARIS (FR)

(Cont. next page)

```
                              1  atgcatcaccatcaccatcat
                                 M  H  H  H  H  H  H
 22 atgagcgccaagtccagaaccatagggattattggagctcctttc
    M  S  A  K  S  R  T  I  G  I  I  G  A  P  F
 67 tcaaagggacagccacgaggaggggtggaagaaggccctacagta
    S  K  G  Q  P  R  G  G  V  E  E  G  P  T  V
112 ttgagaaaggctggtctgcttgagaaacttaaagaacaagagtgt
    L  R  K  A  G  L  L  E  K  L  K  E  Q  E  C
157 gatgtgaaggattatggggacctgccctttgctgacatccctaat
    D  V  K  D  Y  G  D  L  P  F  A  D  I  P  N
202 gacagtccctttcaaattgtgaagaatccaaggtctgtgggaaaa
    D  S  P  F  Q  I  V  K  N  P  R  S  V  G  K
247 gcaagcgagcagctggctggcaaggtggcacaagtcaagaagaac
    A  S  E  Q  L  A  G  K  V  A  Q  V  K  K  N
292 ggaagaatcagcctggtgctgggcggagaccacagtttggcaatt
    G  R  I  S  L  V  L  G  G  D  H  S  L  A  I
337 ggaagcatctctggccatgccagggtccaccctgatcttggagtc
    G  S  I  S  G  H  A  R  V  H  P  D  L  G  V
382 atctgggtggatgctcacactgatatcaacactccactgacaacc
    I  W  V  D  A  H  T  D  I  N  T  P  L  T  T
427 acaagtggaaacttgcatggacaacctgtatctttcctcctgaag
    T  S  G  N  L  H  G  Q  P  V  S  F  L  L  K
472 gaactaaaaggaaagattcccgatgtgccaggattctcctgggtg
    E  L  K  G  K  I  P  D  V  P  G  F  S  W  V
517 actccctgtatatctgccaaggatattgtgtatattggcttgaga
    T  P  C  I  S  A  K  D  I  V  Y  I  G  L  R
562 gacgtggaccctggggaacactacattttgaaaactctaggcatt
    D  V  D  P  G  E  H  Y  I  L  K  T  L  G  I
607 aaatacttttcaatgactgaagtggacagactaggaattggcaag
    K  Y  F  S  M  T  E  V  D  R  L  G  I  G  K
652 gtgatggaagaaacactcagctatctactaggaagaaagaaaagg
    V  M  E  E  T  L  S  Y  L  L  G  R  K  K  R
697 ccaattcatctaagttttgatgttgacggactggacccatctttc
    P  I  H  L  S  F  D  V  D  G  L  D  P  S  F
742 acaccagctactggcacaccagtcgtgggaggtctgacatacaga
    T  P  A  T  G  T  P  V  V  G  G  L  T  Y  R
787 gaaggtctctacatcacagaagaaatctacaaaacagggctactc
    E  G  L  Y  I  T  E  E  I  Y  K  T  G  L  L
832 tcaggattagatataatggaagtgaacccatccctggggaagaca
    S  G  L  D  I  M  E  V  N  P  S  L  G  K  T
877 ccagaagaagtaactcgaacagtgaacacagcagttgcaataacc
    P  E  E  V  T  R  T  V  N  T  A  V  A  I  T
922 ttggcttgtttcggacttgctcgggagggtaatcacaagcctatt
    L  A  C  F  G  L  A  R  E  G  N  H  K  P  I
967 gactaccttaacccacctaagtaa 990
    D  Y  L  N  P  P  K  *
```

Fig. 1B

```
  1 atgagcgccaagtccagaaccatagggattattggagctcctttc
    M  S  A  K  S  R  T  I  G  I  I  G  A  P  F
 46 tcaaagggacagccacgaggaggggtggaagaaggccctacagta
    S  K  G  Q  P  R  G  G  V  E  E  G  P  T  V
 91 ttgagaaaggctggtctgcttgagaaacttaaagaacaagagtgt
    L  R  K  A  G  L  L  E  K  L  K  E  Q  E  C
136 gatgtgaaggattatggggacctgccctttgctgacatccctaat
    D  V  K  D  Y  G  D  L  P  F  A  D  I  P  N
181 gacagtccctttcaaattgtgaagaatccaaggtctgtgggaaaa
    D  S  P  F  Q  I  V  K  N  P  R  S  V  G  K
226 gcaagcgagcagctggctggcaaggtggcacaagtcaagaagaac
    A  S  E  Q  L  A  G  K  V  A  Q  V  K  K  N
271 ggaagaatcagcctggtgctgggcggagaccacagtttggcaatt
    G  R  I  S  L  V  L  G  G  D  H  S  L  A  I
316 ggaagcatctctggccatgccagggtccaccctgatcttggagtc
    G  S  I  S  G  H  A  R  V  H  P  D  L  G  V
361 atctgggtggatgctcacactgatatcaacactccactgacaacc
    I  W  V  D  A  H  T  D  I  N  T  P  L  T  T
406 acaagtggaaacttgcatggacaacctgtatctttcctcctgaag
    T  S  G  N  L  H  G  Q  P  V  S  F  L  L  K
451 gaactaaaaggaaagattcccgatgtgccaggattctcctgggtg
    E  L  K  G  K  I  P  D  V  P  G  F  S  W  V
496 actccctgtatatctgccaaggatattgtgtatattggcttgaga
    T  P  C  I  S  A  K  D  I  V  Y  I  G  L  R
541 gacgtggaccctggggaacactacattttgaaaactctaggcatt
    D  V  D  P  G  E  H  Y  I  L  K  T  L  G  I
586 aaatacttttcaatgactgaagtggacagactaggaattggcaag
    K  Y  F  S  M  T  E  V  D  R  L  G  I  G  K
631 gtgatggaagaaacactcagctatctactaggaagaaagaaaagg
    V  M  E  E  T  L  S  Y  L  L  G  R  K  K  R
676 ccaattcatctaagttttgatgttgacggactggacccatctttc
    P  I  H  L  S  F  D  V  D  G  L  D  P  S  F
721 acaccagctactggcacaccagtcgtgggaggtctgacatacaga
    T  P  A  T  G  T  P  V  V  G  G  L  T  Y  R
766 gaaggtctctacatcacagaagaaatctacaaaacagggctactc
    E  G  L  Y  I  T  E  E  I  Y  K  T  G  L  L
811 tcaggattagatataatggaagtgaacccatccctggggaagaca
    S  G  L  D  I  M  E  V  N  P  S  L  G  K  T
856 ccagaagaagtaactcgaacagtgaacacagcagttgcaataacc
    P  E  E  V  T  R  T  V  N  T  A  V  A  I  T
901 ttggcttgtttcggacttgctcgggagggtaatcacaagcctatt
    L  A  C  F  G  L  A  R  E  G  N  H  K  P  I
946 gactaccttaacccacctaagtaa 969
    D  Y  L  N  P  P  K  *
```

Fig. 1C

## Description

### FIELD OF INVENTION

The present invention is related to pharmaceutical composition and use therefor. In a preferred embodiment, the present invention is related to pharmaceutical composition that is capable to treat hepatitis.

### BACKGROUND OF INVENTION

There are many antiviral drugs for the treatment of hepatitis, the following are the most frequently used: (1) Interferon: a broad-spectrum antiviral agent which induces cells to produce their own antiviral protein through the reaction to the cell surface receptors rather than directly killing or suppressing virus and therefore lead to the suppression of hepatitis B and C virus replication. At the same time it boosts the activity of NK cells, macrophages and T-lymphocytes, modulates immune system and enhances antiviral ability. (2) Interleukin-2: a T-cell growth factor, which modulates immune system and possesses antivirus and anti-tumor ability. (3) Nucleosides: Acyclovir, for example, is an acyclic purine nucleoside which suppresses the replication of various DNA virus. (4) Arabinoside: proved to be potentially effective against hepatitis B both in vivo and in vitro. Some patients show HBV DNA polymerase latency with improved abnormal biochemistry and liver biopsy during treatment. (5) Others: Hepatocyte growth-promoting factor (pHGF), thymosin, anti-hepatitis B ribonucleic acid, ribavirin, levamisole, lentinan, potenline, phytohemagglutinin and etc. However, the effectiveness of the aforesaid drugs is unsatisfying and they are easy to induce adverse side-effect.

### SUMMARY OF INVENTION

In the light of the foregoing background, it is an object of the present invention to provide a more effective pharmaceutical composition for treating hepatitis. In a preferred embodiment, pharmaceutical compositions are provided for selectively reducing arginine level of a patient in the treatment of hepatitis.

Accordingly, in one aspect, an enzyme which degrades arginine (arginine degrading enzyme) is provided for the preparation of medicament. In a preferred embodiment, the arginine degrading enzyme is arginase or arginine deiminase. Yet another embodiment, the arginine degrading enzyme is an isolated and substantially purified recombinant arginase. In a more preferred embodiment, the arginase of the present invention is human arginase I. Yet another more preferred embodiment, the human arginase I of the present invention substantially comprises the same nucleic acid sequence as set forth in SEQ ID NO: 1 or SEQ ID NO: 2. and said nucleic acid sequences comprise the same amino acid sequence as set forth in SEQ ID NO: 3. Yet another embodiment, the recombinant human arginase I of the present invention is of 80-100% purity. In a more preferred embodiment, recombinant human arginase I of the present invention is of 90-100% purity.

Yet another preferred embodiment, the arginase of the present invention is modified to have sufficiently high enzymatic activity and stability to maintain "adequate arginine deprivation" (hereinafter referred to as "AAD") in a patient for at least 3 days. One preferred method of modification is an amino-terminal tag of six-histidine. Yet another preferred modification is pegylation to increase the stability of the enzyme and minimize immunoreactivity elicited by the patient thereto. In another more preferred embodiment, the pegylation comprises a coupling agent covalently bond to at least one polyethylene glycol. In a most preferred embodiment, the coupling agent is 2,4,6-trichloro-s-triazine (cyanuric chloride, CC) or succinimide propionic acid (SPA). The modified arginase has specific activity of at least 250 I.U. /mg. In one preferred embodiment the specific activity is of at least 300-350 I.U. /mg. In a most preferred embodiment, the specific activity is of at least 500 I.U. /mg. In another preferred embodiment, said arginase is modified to have sufficient stability and to have a plasma or serum half-life of at least approximately 3 days.

Yet another preferred embodiment, the medicament prepared by the present invention is provided to treat hepatitis. In a more preferred embodiment, the medicament prepared by the present invention is provided to treat hepatitis B.

In another implementation, there are further provided pharmaceutical composition comprising isolated and substantially purified recombinant arginase. In a preferred embodiment, the pharmaceutical composition provided therein comprising recombinant arginase with 80-100% purity. Yet another preferred embodiment, recombinant human arginase is any arginine degrading enzyme, for example arginine deiminase or human arginase I. In the most preferred embodiment, said enzyme comprises essentially of the same amino acid sequence as set forth in SEQ ID NO: 1 or SEQ ID NO: 2 and said amino acid coding sequences comprise the same amino acid sequence as set forth in SEQ ID NO: 3. In a preferred embodiment, said arginase is modified to have high specific activity and sufficient stability in patient's plasma or serum half-life for approximately 3 days. Another preferred modification is pegylation to increase the stability of the enzyme and minimize immunoreactivity

Yet another aspect of the present invention, a pharmaceutical composition is provided to lower arginine level of a patient. In one preferred embodiment, the present invention is to modulate hepatitis. In a more preferred embodiment, the present invention is capable to treat hepatitis B. In another embodiment, pharmaceutical composition of the present invention is prepared in the form of solid, liquid, emulsion, suspension, small albumin aggregate (SAA) or liposome. Yet another preferred embodiment, the pharmaceutical composition of the present invention is suitable to administrate orally or intravenously.

## BRIEF DESCRIPTION OF FIGURES

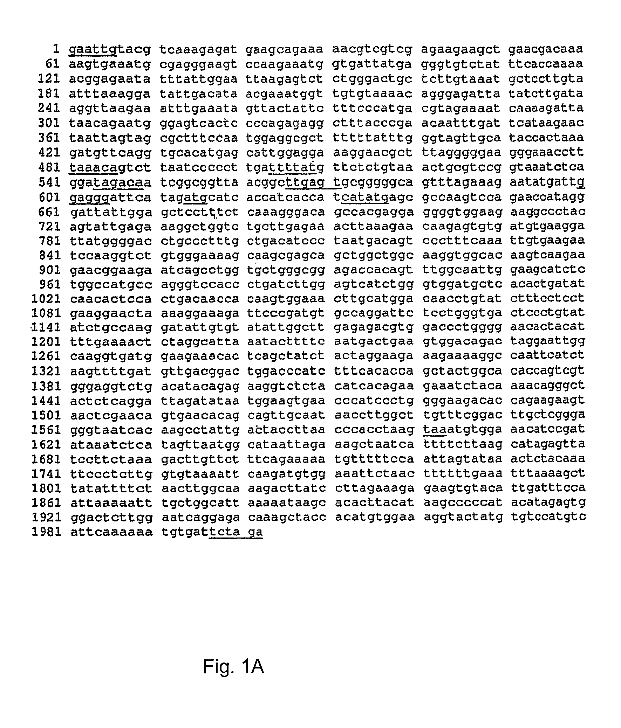

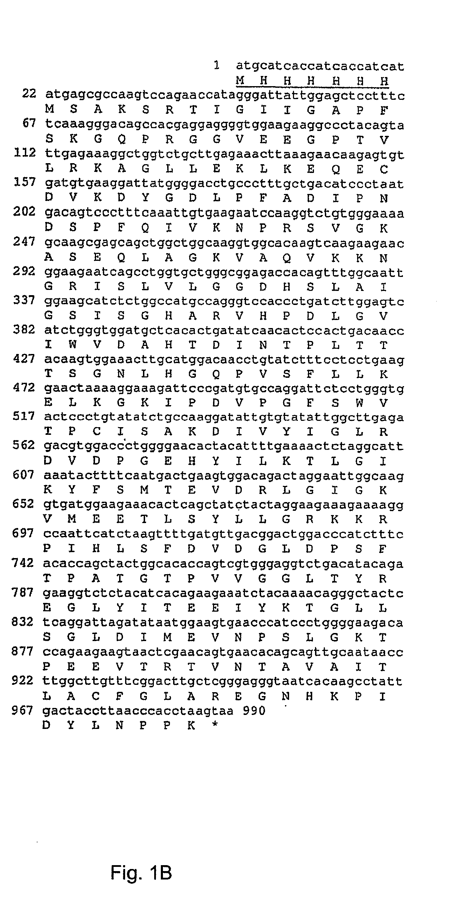

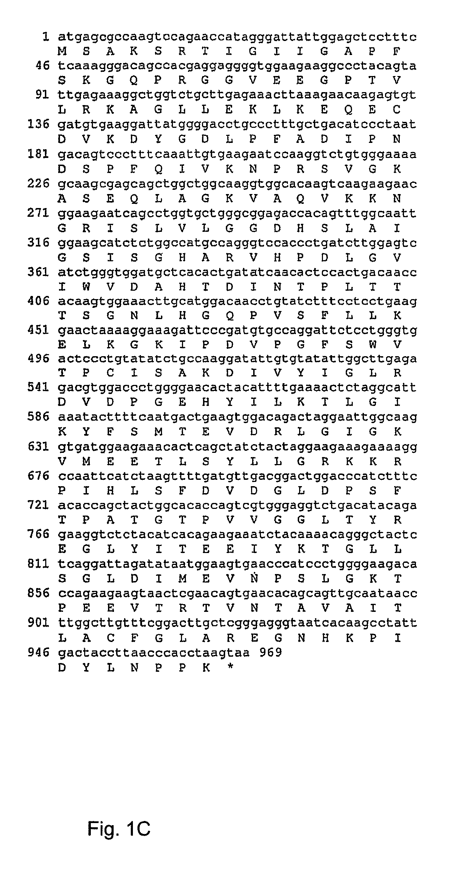

Fig. 1A, 1B and 1C are the nucleic acid sequence of human arginase I and the corresponding amino acid sequence.

Fig. 1A is the nucleic acid sequence (SEQ ID NO: 1) from EcoRI/MunI to XbaI sites of plasmid pAB101. Nucleic acid (nt)1-6, EcoRI/MunI site; nt 481-486, region -35 of promoter 1; nt 504-509, region -10 of promoter 1; nt 544-549, region -35 of promoter 2; nt 566-571, region -10 of promoter 2; nt 600-605, ribosome binding site; nt 614-616, start codon; nt 632-637, NdeI site; nt 1601-1603, stop codon; nt 1997-2002, XbaI site.

Fig. 1B is the nucleic acid sequence (SEQ ID NO: 2) of the modified human arginase and its corresponding amino acid sequence (SEQ ID NO: 3). Nucleic acids 614-1603 in Fig 1A are the coding region of the modified arginase amino acid sequence. The six histidine (SEQ ID NO: 4) on the N-terminal are shown underlined. Translational stop codons are marked with *.

Fig. 1C is the nucleic acid sequence (SEQ ID NO: 8) of normal human arginase I and its corresponding amino acid sequence (SEQ ID NO: 9).

## DETAILED DESCRIPTION OF THE PREFERRED EMBODIMENTS

As used herein, the term "pegylated Arginase" refers to Arginase I of present invention modified by pegylation (see WO2004/001048) to increase the stability of the enzyme and minimize immunoreactivity.

As used herein, the phrase "substantially the same", whether used in reference to the nucleotide sequence of DNA, the ribonucleotide sequence of RNA, or the amino acid sequence of protein, refers to sequences that have slight and non-consequential sequence variations from the actual sequences disclosed herein. Species with sequences that are substantially the same are considered to be equivalent to the disclosed sequences and as such are within the scope of the appended claims. In this regard, "slight and non-consequential sequence variations" means that sequences that are substantially the same as the DNA, RNA, or proteins disclosed and/or claimed herein are functionally equivalent to the sequences disclosed and/or claimed herein. Functionally equivalent sequences will function in substantially the same manner to produce substantially the same compositions as the nucleic acid and amino acid compositions disclosed and claimed herein. In particular, functionally equivalent DNAs encode proteins that are the same as those disclosed herein or proteins that have conservative amino acid variations, such as substitution of a non-polar residue for another non-polar residue or a charged residue for a similarly charged residue. These changes include those recognized by those of skill in the art not to substantially alter the tertiary structure of the protein. The term "sufficiently high enzymatic activity" refers to the enzyme specific activity of the recombinant human arginase for at least 250 I.U./mg, preferably at least 300-350 I.U./mg, more preferably at least 500 I.U./mg. In the preferred embodiment, the arginase has a specific activity of 500-600 I.U./mg. The term "stability" refers to *in vitro* stability of the arginase. More preferably, the stability refers to *in vivo* stability. The rate of decrease of enzyme activity is inversely proportional to the plasma stability of the isolated, purified recombinant human arginase. This relationship is reflected in the half-life of human arginase in plasma.

As used herein, the term "adequate arginine deprivation" (AAD) refers to *in vivo* arginine level at or below 10 $\mu$M. The term "half-life" (1/2-life) refers to the time that would be required for the concentration of the arginase in human plasma *in vitro,* to fall by half.

All other information about the technical know-how and terms as used herein can be found in WO2004/001048 and WO2004/000349.

In order to investigate the anti-hepatitis B virus effect of arginase, the present invention uses hepatitis B viral gene transfected human liver cancer cell line 2.2.15 to test the cellular toxicity of arginase, suppression of HBsAg and HBeAg secretion by arginase and the suppression of HBV-DNA by arginase. Comparison is done using lamivudine by GlaxoWellcome, UK as a positive control. The result shows that: TC50 of pegylated recombinant arginase after 8 days of CPE method drug addition is 40 IU/ml, TC0 is 20$\pm$0 IU/ml. The percent suppression of HBeAg secretion, IC50 and SI from two batches of experiments using TC0 = 20 IU/ml is 68.69$\pm$8.89, 6.37$\pm$0.45 IU/ml, 6.30$\pm$0.45 respectively. The percent suppression of HBsAg secretion, IC50 and SI are 29.81$\pm$27.35, 10.72 IU/ml (from one batch of experiment) and 3.73 (from one batch of experiment) respectively. The IC50 of HBV-DNA dot blotting in the supernatant of the culture medium is 13.18$\pm$0.45 IU/mL, selective index (SI) is 3.19$\pm$0.98. The IC50 of HBV-DNA Southern Blot Sum in cell is 19.79$\pm$7.95 IU/ml, selective index is 2.91$\pm$0.88. The IC50 of HBV-DNA Southern Blot In Lane in cell is 20.06$\pm$1.96 IU/ml, selective index is 2.00$\pm$0.20. The TC50 and TC0 of positive control lamivudine are 1198.97$\pm$97.50 and 800$\pm$0

μg/ml respectively. The HBeAg and HBsAg secretion of 2.2.15 cells are not significantly suppressed after incubating with TC0 800 μg/ml lamivudine for 8 days. The IC50 of HBV-DNA dot blotting in the supernatant of the culture medium is 113.76 μg/mL, selective index is 10.54. The IC50 of HBV-DNA Southern Blot Sum in cell is 88.78±6.37μg/mL, selective index is 13.54±0.97. The multiple experimental results are consistent with published literature, indicating that the experiments are reliable. The result shows that: Arginase significantly inhibits the secretion of HBsAg and HBeAg and lowers HBV-DNA in cells.

Example 1

Preparation of materials

1.1 Drug to be tested

Name: Pegylated recombinant human arginase (BCT-100), hereinafter "arginase". Said arginase comprises nucleic acid sequence as shown in Fig. 1A, 1B and 1C and its corresponding amino acid sequence.

Preparation: Please refer to example 1-8 in specification of WO2004/001048. Recombinant human arginase can be obtained from Professor Ikemoto Masaki's laboratory prior to the earliest application date of WO2004/001048 (University of Kyoto; Address: 53 Kawahara-cho, Shogoin, Sakyo-ku, Kyoto-shi, Kyoto 606-8507 Japan). Arginases are prepared by MEM medium according to the designated dosage groups.

Preservation: store in 4 °C refrigerator.

1.2 Positive control: lamivudine, produced by GlaxoWellcome, UK. Batch No.: B008923, 100 mg per tablet, drug is soaked and dissolved in medium, centrifuge to remove sediments, prepared by MEM medium according to the designated dosage groups during experiment. Preservation: store in 4 °C refrigerator.

1.3 2.2.15 cell: 2.2.15 cell line of human liver cancer cell (Hep G2) transfected with Hepatitis B virus, constructed by Mount Sinai Medical Center. Imported and cultivated by our laboratory.

1.4 Reagents: Eagles MEM powder, G-418 (Geneticin), yeast t-RNA, proteinase-K, by Gibco, U.S.A.; fetal bovine serum, by Hyclone Lab, U.S.A.; L-glutamine, Jingke Chemical Reagent Company; HBsAg, HBeAg radioimmunoassay, China Isotope Corporation Beifang Immunoreagent Research Center; kanamycin, North China Pharmaceutical Group Corporation; polyethylene glycol, Fluka, Sweden; DMSO, Sigma; d-$^{32}$p-dCTP, Beijing Yahui Bio Medical Engineering Company;

1.5 Instruments: culture bottle, Tunclon TM, Denmark; 96-well, 24-well and 6-well plates, Coming, U.S.A.; Carbon Dioxide incubator, Shel-Lab, U.S.A.; γ-counter, Beckman, Germany; Scanner, Microtek; gel-pro analyzer software, MEDIA Cybemetice®;

1.6 Cell culture medium and reagent

MEM medium 100 ml: containing fetal bovine serum 10%, glutamine 0.03%, G418 380 μg/ml, kanamycin 50 μg/ml.

1.7 2.2.15 cell culture: add 0.25% Trypsin into culture bottle with fully grown 2.2.15 cells, digest 10 minutes at 37°C, add medium to disperse, 1:3 subculture, full grown after 10 days.

Example 2

Test for Arginase toxicity to cells

Divide experiment into control group and test groups with different drug concentration. Digest cells, dilute to 200,000 cells/ml, transfer to culture plate, 100 μl per well for 96-well plate, incubate for 24 hours under 5% $CO_2$ at 37°C, ready for experiment when cells grown into monolayer. Dilute arginases with culture medium to 40 IU/ml, serial dilute to 20, 10, 5, 2.5 IU/ml and add into 96-well plates, 5 different concentrations altogether, 3 wells per concentration, change arginase solution every 4 days with the same original concentration. Observe cytopathological changes, 8 days or 4 days under microscope, totally destroyed is 4; 75% destroyed is 3; 50% destroyed is 2, 25% destroyed is 1; no changes is 0. Calculate TC50 and TC0 according to Reed-Muench Method:

$$TC50 = \text{Antilog}\left(B + \frac{50 - B}{A - B} \times C\right)$$

A = log > 50% drug concentration; B = log < 50% drug concentration; C = log times of dilution

Example 3

Test for Arginase suppression of HBeAg and HBsAg

Experiment is designed to have HBsAg and HBeAg positive control group, negative control group, cell control group and test groups with different drug concentration. Grow 200000 cells/ml 2.2.15 cell on 24-well plate, 1ml per well, incubate for 24 hours under 5% $CO_2$ at 37°C. Serial dilute TC0 drug solution into 5 dilutions for each drugs: 20, 10, 5, 2.5, 1.25 IU/ml for Arginase; 800, 400, 200, 100, 50 $\mu$g/ml for lamivudine. 4 wells per concentration, incubate under 5% $CO_2$ at 37°C, change drug solution every 4 days with the same concentration, retrieve culture medium at day 8, preserve at -20 °C. Repeat experiment for 2 batches, test for HBsAg and HBeAg separately. Check cpm value for each wells using $\gamma$-counter.

Calculating drug effectiveness: calculate the mean and standard deviation of cpm from the cell control group and groups with different drug concentration, P/N value and percent suppression, IC50 and SI.

① $$\text{percent antigen suppression (\%)} = \frac{\text{cell control cpm} - \text{cpm of groups with drug}}{\text{cell control cpm}} \text{ X } 100$$

② Calculate IC50 for drug suppression of antigen:

$$\text{IC50} = \text{Antilog (B} + \frac{50 - \text{B}}{\text{A} - \text{B}} \text{ X C)}$$

A = log > 50% drug concentration; B = log < 50% drug concentration; C = log times of dilution

③ SI for Arginase in 2.2.15 cell culture towards HBsAg and HBeAg, calculated according to cellular pathological changes due to cytopathological toxicity.

$$\text{SI} = \frac{\text{Cytopathological toxicity TC50}}{\text{IC50}}$$

④ Calculate the cpm differences between HBsAg and HBeAg in different dilutions and control groups by t-test.

Example 4

Test for Arginase suppression of 2.2.15 cells DNA

Extraction of HBV-DNA from 2.2.15 cells supernatant: Grow 200000 cells/ml 2.2.15 cell on 24-well plate, 1ml per well, add drugs after 24 hours incubation, change drug solution every 4 days with the same concentration, collect supernatant from cell culture after 8 days of incubation counted from the day drugs added into the culture, precipitate with polyethylene glycol, digest with proteinase K, extract with phenol : chloroform : isopentanol, nucleic acid precipitation by absolute ethanol and so on procedures, vacuum dry, re-dissolve in TE buffer as sample.

Dot blot: place dots: take 20 $\mu$l sample (contains 25 $\mu$g DNA), denature, neutralize, serial dilute 20X SSC buffer to 1:8 dilution on nitrocellulose membrane, oven dry, pre-hybridize, hybridize, wash membrane, radioactive self exposure and so on procedures. Develop X ray film with conventional method. Scan developed film with scanner, measure density with gel-pro software, calculate suppression rate and IC50.

$$\text{HBV-DNA suppression in 2.2.15 cell culture medium} = \frac{\text{IOD} - \text{TIOD}}{\text{CIOD}} \text{ X } 100\%$$

Sothern blot: Extraction of HBV-DNA from 2.2.15 cells: add drugs and incubate 2.2.15 cells for 8 days, remove medium and harvest cells, lyse cells with lysis solutions, extracts with equal volume phenol : chloroform : isopentanol twice, add absolute ethanol to precipitate nucleic acid, vacuum dry, re-dissolve in 20 μl TE buffer, add DNA sample buffer, put samples into agarose gel for electrophoresis. After electrophoresis, denature, neutralize and transfer to membrane. Oven dry, hybridize, expose with dot blotting the same time. Scan developed film with scanner, analyze relative density with gel-pro software, and calculate suppression rate and IC50.

Results

Calculate TC50 and TC0 according to Reed-Muench Method. Calculate HBsAg and HBeAg suppression according to above mentioned formulas. Calculate suppression rate and IC50 by analyzing relative density of agarose gel electrophoresis of HBV-DNA.

1. Arginase toxicity in 2.2.15 cell culture

To observe Arginase toxicity towards hepatitis B viral gene transfected human liver cancer 2.2.15 cells, add serial diluted drug solution into the cell culture after 24 hours of incubation. Starting from 40 IU/ml and subsequently 20, 10, 5, 2.5 IU/ml, change drug solution every 4 days until 8 days, observe cytopathological changes under microscope, and check for CPE with microscope. Results: Arginase toxicity in hepatitis B viral gene transfected human liver cancer cell 2.2.15 cells by CPE method (8 days drug administration): TC50 is 40 IU/ml and TC0 is 20±0 μg/ml in two batches experiments. Positive lamivudine control, TC50 is 1198.97±97.50 μg/ml, TC0 is 800±0 μg/ml (see Table. 1A).

2. Arginase suppression of HBeAg and HBsAg

Add Arginase and lamivudine in TC0 concentration into 2.2.15 cells, check cpm value of HBsAg and HBeAg after 8 days, and calculate the effectiveness of drug suppression. See table 2 for experiment results.

2.1. Percent arginase suppression of HBeAg

Two batches Arginase experiments: Serial dilute TC0 20 IU/ml into 10, 5, 2.5 and 1.25 IU/ml, incubate 2.2.15 cells with each concentration for 8 days, average percent suppression of HBeAg in supernatant are: 20 IU/ml, 68.69±8.89% suppression; 10 IU/ml, 60.73±17.49% suppression; 5 IU/ml, 53.96±20.36% suppression; 2.5 IU/ml, 51.83±14.16% suppression; 1.25 IU/ml, 37.34% suppression. Average IC50 is 6.37±0.45 IU/ml, SI is 6.30±0.45.

2.2 Percent arginase suppression of HBsAg

First batch Arginase experiment: The suppression rate of HBsAg in cell culture supernatant of 2.2.15 cell culture after 8 days of incubation with the concentration of 20, 10, 5, 2.5, 1.25 IU/ml are 49.16%, 47.97%, 42.29% and 37.18% respectively. IC50 is 10.72 IU/ml, SI is 3.7.3. However, the percent suppression is low for the second batch. The suppression rate for HBsAg is below 50% with TC0 concentration equals to 20 IU/ml, IC50 > 20 IU/ml.

2.3. The effect of lamivudine on HBsAg and HBeAg

Serial dilute lamivudine from TC0 concentration 800 μg/ml to 400, 200, 100 and 50 μg/ml respectively and add into 2.2.15 cells, check HBsAg and HBeAg titer after 8 days of incubation, calculate the effect of suppression (See table 1B).

2.4. Percent lamivudine suppression of HBeAg

The average percent suppression of HBsAg in cell culture supernatant of 2.2.15 cell culture after 8 days of incubation with lamivudine in the concentration of 800, 400, 200, 100 and 50 μg/ml are: 8.23±3.02%, 12.99±0.46%, 17.83±2.09%, 15.84±2.33%, 14.10±1.27%. No significant suppression is shown.

2.5. Percent lamivudine suppression of HBsAg

The average percent suppression of HBeAg in cell culture supernatant of 2.2.15 cells after 8 days of incubation with lamivudine in the concentration of 800, 400, 200, 100 and 50 μg/ml are: 4.65±6.58%, 4.05±5.73%, 5.67±4.70%, 8.60±4.88%, 3.45±3.95%. No significant suppression is shown.

Table 1A. Arginase Suppression of HBsAg ad HBeAg in 2.2.15 cells (%)

| Drug | Experiment batches | Day of drug addition | Drug concentration | HBeAg (CPM) | | | HBsAg (CPM) | | |
|---|---|---|---|---|---|---|---|---|---|
| | | | | % suppression | IC50 IU/ml | SI | % suppression | IC50 IU/ml | SI |
| Arginase | 1 | 8 | 20 | 74.9808 | 6.69 | 5.98 | 49.1558 | 10.72 | 3.73 |
| | | | 10 | 73.0985 | | | 47.9651 | | |
| | | | 5 | 68.3484 | | | 42.2871 | | |
| | | | 2.5 | 61.8387 | | | 37.1787 | | |
| | 2 | 8 | 20 | 62.4033 | 6.05 | 6.61 | 10.4731 | > 20.00 | |
| | | | 10 | 48.3596 | | | 6.0761 | | |
| | | | 5 | 39.5618 | | | 1.4739 | | |
| | | | 2.5 | 41.8149 | | | 2.6073 | | |
| | | | 1.25 | 37.3426 | | | 4.463 | | |
| | Two batches average | | 20 | 68.69±8.89 | 6.37 ±0.45 | 6.30 ±0.45 | 29.81±27.35 | 1st batch 10.72 2nd batch >20 Not even | |
| | | | 10 | 60.73±17.49 | | | 27.02±29.62 | | |
| | | | 5 | 53.96±20.36 | | | 21.88±28.86 | | |
| | | | 2.5 | 51.83±14.16 | | | 19.92±24.40 | | |
| | | | 1.25 | 37.34 | | | 4.46 | | |

Table 1B. Lamivudine Suppression of HBsAg ad HBeAg in 2.2.15 cells (%)

| Drug | Experiment batches | Day of drug addition | Drug concentrationµg/ml | HBeAg (CPM) % suppression | HBeAg (CPM) IC50 µg/ml | HBeAg (CPM) SI | HBsAg (CPM) % suppression | HBsAg (CPM) IC50µg/ml | HBsAg (CPM) SI |
|---|---|---|---|---|---|---|---|---|---|
| Lamivudine | 1 | 8 | 800 | 6.10 | >800 | | 0 | >800 | |
| | | | 400 | 12.67 | | | 8.10 | | |
| | | | 200 | 16.35 | | | 2.35 | | |
| | | | 100 | 17.48 | | | 12.05 | | |
| | | | 50 | 13.20 | | | 6.24 | | |
| | 2 | 8 | 800 | 10.37 | >800 | | 9.299 | >800 | |
| | | | 400 | 13.32 | | | 0 | | |
| | | | 200 | 19.31 | | | 8.99 | | |
| | | | 100 | 14.19 | | | 5.15 | | |
| | | | 50 | 14.99 | | | 0.65 | | |
| | Two batches average | 8 | 800 | 8.23±3.02 | >800±0 TC0 800 µg/ml. No significant suppression shown. | | 4.65±6.58 | >800±0 TC0 800 µg/ml. No significant suppression shown. | |
| | | | 400 | 12.99±0.46 | | | 4.05±5.73 | | |
| | | | 200 | 17.83±2.09 | | | 5.67±4.7 | | |
| | | | 100 | 15.84±2.33 | | | 8.6±4.88 | | |
| | | | 50 | 14.10±1.27 | | | 3.45±3.95 | | |

3. Arginase and lamivudine suppression of HBV-DNA in supernatant of 2.2.15 cell culture

3.1 Arginase dot blotting in HBV-DNA in supernatant of 2.2.15 cell culture

The effect of arginase on HBV-DNA in supernatant of 2.2.15 cell culture, the IC50 of two batches of test solution against HBV-DNA after 8 days of incubation are 16.04, 10.31 IU/ml. average IC50 is 13.18±4.05 IU/ml, SI are 2.49, 3.88, average is 3.19± 0.98. See Table 2 for result.

Table 2. Effect of Arginase on HBV-DNA in supernatant of 2.2.15 cell culture

| Batch | Day of drug addition | Drug concentration (μg/ml) | Dilution factor/percent suppression of HBV-DNA in cell culture supernatant | | | |
|---|---|---|---|---|---|---|
| | | | Original Solution (IOD) | % suppression | IC50 (μg/ml) | SI |
| 1 | 8 | 20 | 1643.3 | 30.8113 | 16.04 | 2.49 |
| | | 10 | 1680.6 | 29.2409 | | |
| | | 5 | 2090.38 | 11.9877 | | |
| | | 2.5 | 1783.34 | 24.9152 | | |
| | | Control | 2375.1 | | | |
| 2 | 8 | 20 | 2430.14 | 47.7577 | 10.31 | 3.88 |
| | | 10 | 2881.48 | 38.0549 | | |
| | | 5 | 2613.11 | 43.8243 | | |
| | | 2.5 | 4118.31 | 11.466 | | |
| | | 1.25 | 3917.78 | 15.7769 | | |
| | | Control | 4651.67 | | | |
| Two batches average | | | | | 13.18±4.05 | 3.19±0.98 |

3.2 Effect of lamivudine to HBV-DNA in supernatant of 2.2.15 cell culture

The effect of lamivudine to HBV-DNA in supernatant of 2.2.15 cell culture of first batch experiment: IC50 is 113.76 μg/ml, TC50 is 1198.97±97.50 μg/ml, SI is 10.54. See result in Table 3.

| Drug | Day of drug addition | Drug concentration (μg/ml) | Dilution factor/percent suppression of HBV-DNA in cell culture supernatant | | | |
|---|---|---|---|---|---|---|
| | | | CPM | % suppression | IC50 (μg/ml) | SI |
| Lamivudine | 8 | 800 | 663.013 | 82.5905 | 113.76 | 10.54 |
| | | 400 | 795.628 | 79.1083 | | |
| | | 200 | 1080.03 | 71.6404 | | |
| | | 100 | 1465.31 | 61.5237 | | |
| | | 50 | 2831.21 | 25.6576 | | |
| | | Control | 3808.34 | | | |

Table 3. Effect of Lamivudine on HBV-DNA in supernatant of 2.2.15 cell culture

3.3. Arginase and lamivudine suppression of HBV-DNA Southern Blot in 2.2.15 cells

3.3.1 The suppression of HBV-DNA Southern Blot in 2.2.15 cell by Arginase

Results show: The result of total HBV-DNA Southern Blot in 2.2.15 cells is totally suppressed after 8 days of incubation with Arginase added: two batches of experiment IC50 are 25.42, 14.17 IU/ml, average IC50 is 19.79±7.95, SI are 1.57 and 2.82 respectively, average is 2.19±0.88. The result of total HBV-DNA Southern Blot In Lane in 2.2.15 cells: two batches of experiment IC50 are 21.45, 18.67 IU/ml, average is 20.06±1.96 IU/ml, SI are 1.86, 2.14, average is 2.00±0.20. See results in Table 4.

Table 4. Arginase suppression of HBV-DNA Southern Blot in 2.2.15 cells

| Batch | Day of drug | Drug concentration | Sum (IOD) | % Suppression | IC50 µg/ml | SI | In Lane (IOD) | % Suppression | IC50 IU/ml | SI |
|---|---|---|---|---|---|---|---|---|---|---|
| 1 | 8 | 20 | 25011 | 14.9893 | 25.42 | 1.57 | 31436 | 25.2982 | 21.45 | 1.86 |
| | | 10 | 24234 | 17.6303 | | | 32499 | 22.7722 | | |
| | | 5 | 20104 | 31.6679 | | | 28796 | 31.5717 | | |
| | | 2.5 | 21650 | 26.4131 | | | 28762 | 31.6525 | | |
| | | Control | 29421 | | | | 42082 | | | |
| 2 | 8 | 20 | 34433 | 47.9416 | 14.17 | 2.82 | 46760 | 38.6255 | 18.67 | 2.14 |
| | | 10 | 46884 | 29.1172 | | | 66241 | 13.0559 | | |
| | | 5 | 46283 | 30.0259 | | | 61655 | 19.0752 | | |
| | | 2.5 | 68619 | 0 | | | 88350 | 38.6255 | | |
| | | Control | 66143 | | | | 76188 | | | |

3.3.2 The suppression of HBV-DNA Southern Blot in 2.2.15 cells by lamivudine

Results show: The suppression effect of total HBV-DNA Southern Blot in 2.2.15 cells with lamivudine: two batches of experiment IC50 are 84.27, 93.28µg /ml, average is 88.78±6.37µg/ml, TC50 is 1198.97 µg/ml, SI are 14.23 and 12.85 respectively, average is 13.54±0.97 (see Table 5).

Table 5. Lamivudine suppression of HBV-DNA Southern Blot in 2.2.15 cells

| Batch | Day of drug addition | Drug concentration μg/ml | Sum (IOD) | % Suppression | IC50 μg/ml | SI |
|---|---|---|---|---|---|---|
| 1 | 8 | 800 μg/ml | 143.91 | 90.50 | 84.27 | 14.23 |
| | | 400 | 317.332 | 70.04 | | |
| | | 200 | 366.35 | 75.80 | | |
| | | 100 | 491.77 | 67.52 | | |
| | | 50 | 748.91 | 50.54 | | |
| | | Control | 1514.08 | | | |
| 2 | 8 | 800 μg/ml | 509.85 | 79.01 | 93.28 | 12.85 |
| | | 400 | 804.63 | 66.87 | | |
| | | 200 | 589.01 | 75.75 | | |
| | | 100 | 1002.21 | 58.74 | | |
| | | 50 | 710.239 | 70.76 | | |
| | | Control | 2428.92 | | | |
| Two batches average | | | | | 88.78±6.37 | 13.54±0.97 |

Discussion

The experiment observes the toxicity of Arginase and anti-hepatitis B virus positive control drug lamivudine on hepatitis B virus transfected human liver cancer cell 2215 cell line after 8 days of added drug incubation, the suppression of HBsAg and HBeAg secretion and in cell culture supernatant, and the suppression of HBV-DNA in cells. See Table 6 for summary.

Table 6. Summary of Effect of Arginase and Lamivudine on HBV-DNA in 2.2.15 cells

| Drugs | Cellular toxicity IU/ml | | HBeAg | | HBsAg | | Cell Supernatant HBV-DNA | | Cell HBV-DNA Southern Blot | | | |
|---|---|---|---|---|---|---|---|---|---|---|---|---|
| | TC50 | TC0 | IC50 IU/ml | SI | IC50 IU/ml | SI | IC50 IU/ml | SI | IC50 μg/ml | SI | IC50 μg/ml | SI |
| Arginase | 40 | 20±0 | 6.37±0.45 | 6.30±0.45 | ①10.72 ②>20 | ①3.73 ②≤1 | 13.18±4.05 | 3.19 ± 0.98 | 19.79 ±7.95 | 2.19± 0.88 | 20.6±1.96 | 2.00± 0.20 |
| Lamivudine | 1198.97± 97.50 | 800± 0 μg/ml | >800 μg/ml | | | | >800 μg/ml | | 113.76 | 10.54 | 88.78± 6.37 | 13.54± 0.97 |

Annotation: ① first batch, ② second batch

1. Arginase toxicity to 2.2.15 cells

TC50 of Arginase is 40 IU/ml, TC0 is 20±0 IU/ml.

TC50 of positive control lamivudine is 1198.97±97.50 μg/ml; TC0 is 800±0 μg/ml.

2. Arginase and lamivudine suppression of the secretion of HBsAg and HBeAg in 2.2.15 cells

Serial dilute 4 concentrations of TC0 20 IU/ml Arginase and added into 2.2.15 cells to incubate for 8 days, the average suppression rate of two batches of experiments on HBeAg secretion is 68.69±8.89%, the IC50 to HBeAg is 6.37±0.45 IU/ml, SI is 6.30±0.45. The suppression rate of HBsAg is 29.81±27.35%, the IC50 to HBsAg are: first batch 10.72 IU/ml, SI is 3.73, second batch is 20 IU/ml.

Suppression rate is below 50%, IC50 >20 IU/ml, SI:≤ 1. No average has been taken for the two batches of experiments.

No significant suppression action for HBeAg and HBsAg by adding TC0 800 μg/ml of lamivudine into 2.2.15 cell culture to incubate for 8 days. Half of the effective concentration and SI cannot be calculated.

3. Arginase and lamivudine suppression of HBV-DNA in 2.2.15 cells

Results show: The IC50 of Arginase in HBV-DNA Dot Blot from supernatant of cell culture added with drug after 8 days of incubation is 13.18±4.05 IU/ml, SI is 3.19±0.98. The IC50 in HBV-DNA Southern Blot after 8 days is 19.79±7.95 IU/ml, SI is 2.19±0.88. The IC50 in HBV-DNA Dot Blot with added drug In Lane after 8 days is 20.06±1.96 μg/ml, SI is 2.00±0.20.

The IC50 of lamivudine in HBV-DNA Dot Blot is 113.76 μg/ml, SI is 10.54. In suppression of Southern blot, the IC50 of both batches of experiments are 84.27 and 93.28 μg/ml, average is 88.78±6.37 μg/ml, TC50 is 1198.97 μg/ml, SI are 14.23 and 12.85 respectively, average is 13.54±0.97

It must be noted that as used herein and in the appended claims, the singular forms "a" and "the" include plural references unless the context clearly dictates otherwise. Thus, for example, reference to "a pharmaceutical preparation" includes mixtures of different preparations and reference to "the method of treatment" includes reference to equivalent steps and methods known to those skilled in the art, and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described. All publications mentioned herein are incorporated herein by reference to describe and disclose specific information for which the reference was cited in connection with. The invention having been fully described, modifications within its scope will be apparent to those of ordinary skill in the art. All such modifications are within the scope of the invention.

Formulations of the pharmaceutical composition of the present invention can be used in the form of a solid, a solution, an emulsion, a dispersion, a micelle, a liposome, and the like, wherein the resulting formulation contains one or more of the modified human arginase in the practice of the present invention, as active ingredients, in a mixture with an organic or inorganic carrier or excipient suitable for enteral or parenteral applications. The active ingredients may be the arginase, for example, with the usual non-toxic, pharmaceutically acceptable carriers for tablets, pellets, capsules, suppositories, solutions, emulsions, suspensions, and any other form suitable for use in manufacturing preparations, in solid, semisolid, or liquid form. In addition auxiliary, stabilizing, thickening and coloring agents and perfumes may be used. The active ingredients of one or more arginase are included in the pharmaceutical formulation in an amount sufficient to produce the desired effect upon the target process, condition or disease.

Pharmaceutical formulations containing the active ingredients contemplated herein may be in a form suitable for oral use, for example, as tablets, troches, lozenges, aqueous or oily suspensions, dispersible powders or granules, emulsions, hard or soft capsules, or syrups or elixirs. Formulations intended for oral use may be prepared according to any method known in the art for the manufacture of pharmaceutical formulations. The tablets may be uncoated or they may be coated by known techniques to delay disintegration and absorption in the gastrointestinal tract, thereby providing sustained action over a longer period. They may also be coated to form osmotic therapeutic tablets for controlled release.

In some cases, formulations for oral use may be in the form of hard gelatin capsules wherein the active ingredients are mixed with an inert solid diluent, for example, calcium carbonate, calcium phosphate, kaolin, or the like. They may also be in the form of soft gelatin capsules wherein the active ingredients are mixed with water or an oil medium, for example, peanut oil, liquid paraffin, or olive oil.

The pharmaceutical formulations may also be in the form of a sterile injectable solution or suspension. This suspension may be formulated according to known methods using suitable dispersing or wetting agents and suspending agents. The sterile injectable preparation may also be a sterile injectable solution or suspension in a non-toxic parenterally-acceptable diluent or solvent, for example, as a solution in 1,4-butanediol. Sterile, fixed oils are conventionally employed as a solvent or suspending medium. For this purpose any bland fixed oil may be employed including synthetic mono- or diglycerides, fatty acids (including oleic acid), naturally occurring vegetable oils like sesame oil, coconut oil, peanut oil, cottonseed oil, or synthetic fatty vehicles, like ethyl oleate, or the like. Buffers, dextrose solutions preservatives, antioxidants, and the like, can be incorporated or used as solute to dissolve the soluble enzyme as required.

The pharmaceutical formulations may also be an adjunct treatment together with other chemotherapeutic agents.

**Claims**

1. The use of an arginine degrading enzyme in the manufacture of a medicament for the treatment of hepatitis.

2. The use according to claim 1, wherein said enzyme is an isolated and substantially purified recombinant arginase.

3. The use according to claim 1, wherein the purity of said recombinant arginase is 80-100%.

4. The use according to claim 3, wherein said recombinant arginase is human arginase I.

5. The use according to claim 3, wherein said recombinant arginase is arginine deiminase.

6. The use according to claim 4, wherein said enzyme comprising substantially the same nucleic acid sequence as set forth in SEQ ID NO: 1 or SEQ ID NO: 2, wherein said nucleic acid sequence comprising substantially the same amino acid sequence as set forth in SEQ ID NO: 3.

7. The use according to claim 4, wherein said enzyme having a specific activity of 250 I.U./mg.

8. The use according to claim 4, wherein said enzyme comprising a modification that results in having sufficient stability and an in vitro plasma half-life of at least approximately 3 days.

9. The use according to claim 8, wherein said enzyme is pegylated.

10. The use according to claim 9, wherein said pegylation results from covalently attaching at least one polyethylene glycol (PEG) moiety to said arginase using a coupling agent.

11. The use according to claim 9, wherein said coupling agent is 2,4,6-trichloro-s-triazine (cyanuric chloride, CC) or succinimide propionic acid (SPA).

12. The use according to claim 4, wherein said human arginase I comprising six histidines attached to the amino terminal end thereof.

13. The use according to claim 1, wherein said hepatitis is hepatitis B.

14. A pharmaceutical composition comprising arginine degrading enzyme.

15. The pharmaceutical composition of claim 14, wherein said enzyme is an isolated and substantially purified recombinant arginase.

16. The pharmaceutical composition of claim 15, wherein the purity of said recombinant arginase is 80-100%.

17. The pharmaceutical composition of claim 15, wherein said recombinant arginase is human arginase I.

18. The pharmaceutical composition of claim 15, wherein said recombinant arginase is arginine deiminase.

19. The pharmaceutical composition of claim 17, wherein said enzyme comprising substantially the same nucleic acid sequence as set forth in SEQ ID NO: 1 or SEQ ID NO: 2, wherein said nucleic acid sequence comprising substantially the same amino acid sequence as set forth in SEQ ID NO: 3.

20. The pharmaceutical composition of claim 17, wherein said enzyme having a specific activity of 250 I.U./mg.

21. The pharmaceutical composition of claim 17, wherein said enzyme having a half-life of at least 3 days in patient plasma.

22. The pharmaceutical composition of claim 17, wherein said enzyme having a half-life of at least 1 days in patient plasma.

23. The pharmaceutical composition of claim 17 wherein said enzyme is modified by pegylation.

24. The pharmaceutical composition of claim 17, wherein said human arginase I comprising six histidines attached to the amino terminal end thereof.

25. The pharmaceutical composition of claim 14, wherein said enzyme reduces the physiological arginine level in patients.

26. The pharmaceutical composition of claim 14, wherein said enzyme modulates hepatitis.

**27.** The pharmaceutical composition of claim 24, wherein said hepatitis is hepatitis B.

**28.** The pharmaceutical composition of claim 14, wherein said composition can be further manufactured in the form of a solid, a solution, an emulsion, a dispersion, a micelle, or a liposome.

**29.** The pharmaceutical composition of claim 14, wherein said composition is suitable for oral use or injection.

```
   1 gaattgtacg tcaaagagat gaagcagaaa aacgtcgtcg agaagaagct gaacgacaaa
  61 aagtgaaatg cgagggaagt ccaagaaatg gtgattatga gggtgtctat ttcaccaaaa
 121 acggagaata tttattggaa ttaagagtct ctgggactgc tcttgtaaat gctccttgta
 181 atttaaagga tattgacata acgaaatggt tgtgtaaaac agggagatta tatcttgata
 241 aggttaagaa atttgaaata gttactattc tttcccatga cgtagaaaat caaaagatta
 301 taacagaatg ggagtcactc cccagagagg ctttacccga acaatttgat tcataagaac
 361 taattagtag cgctttccaa tggaggcgct tttttatttg ggtagttgca taccactaaa
 421 gatgttcagg tgcacatgag cattggagga aaggaacgct ttagggggaa gggaaacctt
 481 taaacagtct taatccccct tgattttatg ttctctgtaa actgcgtccg gtaaatctca
 541 ggatagacaa tcggcggtta acggcttgag tgcgggggca gtttagaaag aatatgattg
 601 gagggattca tagatgcatc accatcacca tcatatgagc gccaagtcca gaaccatagg
 661 gattattgga gctcctttct caaagggaca gccacgagga ggggtggaag aaggccctac
 721 agtattgaga aaggctggtc tgcttgagaa acttaaagaa caagagtgtg atgtgaagga
 781 ttatggggac ctgccctttg ctgacatccc taatgacagt ccctttcaaa ttgtgaagaa
 841 tccaaggtct gtgggaaaag caagcgagca gctggctggc aaggtggcac aagtcaagaa
 901 gaacggaaga atcagcctgg tgctgggcgg agaccacagt ttggcaattg gaagcatctc
 961 tggccatgcc agggtccacc ctgatcttgg agtcatctgg gtggatgctc acactgatat
1021 caacactcca ctgacaacca caagtggaaa cttgcatgga caacctgtat ctttcctcct
1081 gaaggaacta aaggaaaga ttcccgatgt gccaggattc tcctgggtga ctccctgtat
1141 atctgccaag gatattgtgt atattggctt gagagacgtg gaccctgggg aacactacat
1201 tttgaaaact ctaggcatta aatacttttc aatgactgaa gtggacagac taggaattgg
1261 caaggtgatg gaagaaacac tcagctatct actaggaaga aagaaaaggc caattcatct
1321 aagttttgat gttgacggac tggacccatc tttcacacca gctactggca caccagtcgt
1381 gggaggtctg acatacagag aaggtctcta catcacagaa gaaatctaca aaacagggct
1441 actctcagga ttagatataa tggaagtgaa cccatccctg ggaagacac cagaagaagt
1501 aactcgaaca gtgaacacag cagttgcaat aaccttggct tgtttcggac ttgctcggga
1561 gggtaatcac aagcctattg actaccttaa cccacctaag taaatgtgga aacatccgat
1621 ataaatctca tagttaatgg cataattaga aagctaatca ttttcttaag catagagtta
1681 tccttctaaa gacttgttct ttcagaaaaa tgtttttcca attagtataa actctacaaa
1741 ttccctcttg gtgtaaaatt caagatgtgg aaattctaac tttttgaaa tttaaaagct
1801 tatattttct aacttggcaa aagacttatc cttagaaaga gaagtgtaca ttgatttcca
1861 attaaaaatt tgctggcatt aaaaataagc acacttacat aagcccccat acatagagtg
1921 ggactcttgg aatcaggaga caaagctacc acatgtggaa aggtactatg tgtccatgtc
1981 attcaaaaaa tgtgattcta ga
```

Fig. 1A

```
                     1  atgcatcaccatcaccatcat
                        M  H  H  H  H  H  H
 22 atgagcgccaagtccagaaccatagggattattggagctcctttc
    M  S  A  K  S  R  T  I  G  I  I  G  A  P  F
 67 tcaaagggacagccacgaggaggggtggaagaaggccctacagta
    S  K  G  Q  P  R  G  G  V  E  E  G  P  T  V
112 ttgagaaaggctggtctgcttgagaaacttaaagaacaagagtgt
    L  R  K  A  G  L  L  E  K  L  K  E  Q  E  C
157 gatgtgaaggattatggggacctgccctttgctgacatccctaat
    D  V  K  D  Y  G  D  L  P  F  A  D  I  P  N
202 gacagtccctttcaaattgtgaagaatccaaggtctgtgggaaaa
    D  S  P  F  Q  I  V  K  N  P  R  S  V  G  K
247 gcaagcgagcagctggctggcaaggtggcacaagtcaagaagaac
    A  S  E  Q  L  A  G  K  V  A  Q  V  K  K  N
292 ggaagaatcagcctggtgctgggcggagaccacagtttggcaatt
    G  R  I  S  L  V  L  G  G  D  H  S  L  A  I
337 ggaagcatctctggccatgccagggtccaccctgatcttggagtc
    G  S  I  S  G  H  A  R  V  H  P  D  L  G  V
382 atctgggtggatgctcacactgatatcaacactccactgacaacc
    I  W  V  D  A  H  T  D  I  N  T  P  L  T  T
427 acaagtggaaacttgcatggacaacctgtatctttcctcctgaag
    T  S  G  N  L  H  G  Q  P  V  S  F  L  L  K
472 gaactaaaaggaaagattcccgatgtgccaggattctcctgggtg
    E  L  K  G  K  I  P  D  V  P  G  F  S  W  V
517 actccctgtatatctgccaaggatattgtgtatattggcttgaga
    T  P  C  I  S  A  K  D  I  V  Y  I  G  L  R
562 gacgtggaccctggggaacactacatttgaaaactctaggcatt
    D  V  D  P  G  E  H  Y  I  L  K  T  L  G  I
607 aaatacttttcaatgactgaagtggacagactaggaattggcaag
    K  Y  F  S  M  T  E  V  D  R  L  G  I  G  K
652 gtgatggaagaaacactcagctatctactaggaagaaagaaaagg
    V  M  E  E  T  L  S  Y  L  L  G  R  K  K  R
697 ccaattcatctaagttttgatgttgacggactggacccatctttc
    P  I  H  L  S  F  D  V  D  G  L  D  P  S  F
742 acaccagctactggcacaccagtcgtgggaggtctgacatacaga
    T  P  A  T  G  T  P  V  V  G  G  L  T  Y  R
787 gaaggtctctacatcacagaagaaatctacaaaacagggctactc
    E  G  L  Y  I  T  E  E  I  Y  K  T  G  L  L
832 tcaggattagatataatggaagtgaacccatccctggggaagaca
    S  G  L  D  I  M  E  V  N  P  S  L  G  K  T
877 ccagaagaagtaactcgaacagtgaacacagcagttgcaataacc
    P  E  E  V  T  R  T  V  N  T  A  V  A  I  T
922 ttggcttgtttcggacttgctcgggagggtaatcacaagcctatt
    L  A  C  F  G  L  A  R  E  G  N  H  K  P  I
967 gactaccttaacccacctaagtaa 990
    D  Y  L  N  P  P  K  *
```

Fig. 1B

```
  1 atgagcgccaagtccagaaccatagggattattggagctcctttc
    M  S  A  K  S  R  T  I  G  I  I  G  A  P  F
 46 tcaaagggacagccacgaggaggggtggaagaaggccctacagta
    S  K  G  Q  P  R  G  G  V  E  E  G  P  T  V
 91 ttgagaaaggctggtctgcttgagaaacttaaagaacaagagtgt
    L  R  K  A  G  L  L  E  K  L  K  E  Q  E  C
136 gatgtgaaggattatggggacctgccctttgctgacatccctaat
    D  V  K  D  Y  G  D  L  P  F  A  D  I  P  N
181 gacagtccctttcaaattgtgaagaatccaaggtctgtgggaaaa
    D  S  P  F  Q  I  V  K  N  P  R  S  V  G  K
226 gcaagcgagcagctggctggcaaggtggcacaagtcaagaagaac
    A  S  E  Q  L  A  G  K  V  A  Q  V  K  K  N
271 ggaagaatcagcctggtgctgggcggagaccacagtttggcaatt
    G  R  I  S  L  V  L  G  G  D  H  S  L  A  I
316 ggaagcatctctggccatgccagggtccaccctgatcttggagtc
    G  S  I  S  G  H  A  R  V  H  P  D  L  G  V
361 atctgggtggatgctcacactgatatcaacactccactgacaacc
    I  W  V  D  A  H  T  D  I  N  T  P  L  T  T
406 acaagtggaaacttgcatggacaacctgtatctttcctcctgaag
    T  S  G  N  L  H  G  Q  P  V  S  F  L  L  K
451 gaactaaaaggaaagattcccgatgtgccaggattctcctgggtg
    E  L  K  G  K  I  P  D  V  P  G  F  S  W  V
496 actccctgtatatctgccaaggatattgtgtatattggcttgaga
    T  P  C  I  S  A  K  D  I  V  Y  I  G  L  R
541 gacgtggaccctggggaacactacattttgaaaactctaggcatt
    D  V  D  P  G  E  H  Y  I  L  K  T  L  G  I
586 aaatacttttcaatgactgaagtggacagactaggaattggcaag
    K  Y  F  S  M  T  E  V  D  R  L  G  I  G  K
631 gtgatggaagaaacactcagctatctactaggaagaaagaaaagg
    V  M  E  E  T  L  S  Y  L  L  G  R  K  K  R
676 ccaattcatctaagttttgatgttgacggactggacccatctttc
    P  I  H  L  S  F  D  V  D  G  L  D  P  S  F
721 acaccagctactggcacaccagtcgtgggaggtctgacatacaga
    T  P  A  T  G  T  P  V  V  G  G  L  T  Y  R
766 gaaggtctctacatcacagaagaaatctacaaaacagggctactc
    E  G  L  Y  I  T  E  E  I  Y  K  T  G  L  L
811 tcaggattagatataatggaagtgaacccatccctggggaagaca
    S  G  L  D  I  M  E  V  N  P  S  L  G  K  T
856 ccagaagaagtaactcgaacagtgaacacagcagttgcaataacc
    P  E  E  V  T  R  T  V  N  T  A  V  A  I  T
901 ttggcttgtttcggacttgctcgggagggtaatcacaagcctatt
    L  A  C  F  G  L  A  R  E  G  N  H  K  P  I
946 gactaccttaacccacctaagtaa 969
    D  Y  L  N  P  P  K  *
```

Fig. 1C

# INTERNATIONAL SEARCH REPORT

International application No.
PCT/CN2005/001411

A. CLASSIFICATION OF SUBJECT MATTER

IPC7:A61K38/46,A61P1/16

According to International Patent Classification (IPC) or to both national classification and IPC

B. FIELDS SEARCHED

Minimum documentation searched (classification system followed by classification symbols)

IPC7: A61K38, A61P1

Documentation searched other than minimum documentation to the extent that such documents are included in the fields searched

CNKI, MEDLINE, CA

Electronic data base consulted during the international search (name of data base and, where practicable, search terms used)

CNPAT, CNKI and keywords: ARGINASE, HEPATITIS
WPI, EPODOC, PAJ, MEDLINE, CA and keywords: ARGINASE, HEPATITIS
GENEBANK: sequence search based on SEQ ID NO: 3

C. DOCUMENTS CONSIDERED TO BE RELEVANT

| Category* | Citation of document, with indication, where appropriate, of the relevant passages | Relevant to claim No. |
|---|---|---|
| X | WO2004001048A1 (BIO-CANCER TREATMENT INTERNATIONAL LIMITED (CHINA)) 31.DEC 2003 (31.12.2003) claims, sequences list. | 15-17,19-24 |
| A | CN1140588A (HAO,Wenxue (CHINA)) 22.JAN 1997 (22.01.1997) all the text | 2-4,6-13 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 16.NOV 2005(16.11.2005) | 08 · DEC 2005 (08 · 12 · 2005) |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br>KE,Ke<br>Telephone No. (86-10)62085090 |

Form PCT/ISA /210 (second sheet) (April 2005)

# INTERNATIONAL SEARCH REPORT

International application No.
PCT/CN2005/001411

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 1, 5, 14, 18, 25-29
because they relate to parts of the international application that do not comply with the prescribed requirements to such an extent that no meaningful international search can be carried out, specifically:
these claims relate to the use or pharmaceutical compositions of arginine degradative enzyme or arginine deiminase.However the tests in description are only based on arginase.These enzymes don't have just the same character as that of arginase. So the results of these enzymes can't be deduced from the description.Thus claims 1,5,14,18,25-29 are not supported by the description and don't meet the criterion mentioned in article 6 PCT.

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fees, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA /210 (continuation of first sheet (2)) (April 2005)

**INTERNATIONAL SEARCH REPORT**
Information on patent family members

International application No.
PCT/CN2005/001411

| Patent Documents referred in the Report | Publication Date | Patent Family | Publication Date |
|---|---|---|---|
| WO2004001048 | 31.12.2003 | AU2002325782 A1 | 06.01.2004 |

Form PCT/ISA /210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004001048 A
- WO 2004000349 A

SEQUENCE LISTING

<110> Bio-Cancer Treatment International Limited

<120> Pharmaceutical Composition and Method of Treating Hepatitis with Arginases

<130> P046439EP

<140> EP 05783950.8
<141> 2005-09-06

<150> CN 2004/0076854.4
<151> 2004-09-08

<160> 9

<170> PatentIn version 3.2

<210> 1
<211> 2002
<212> DNA
<213> Homo sapiens

<400> 1
```
gaattgtacg tcaaagagat gaagcagaaa aacgtcgtcg agaagaagct gaacgacaaa     60

aagtgaaatg cgagggaagt ccaagaaatg gtgattatga gggtgtctat ttcaccaaaa    120

acggagaata tttattggaa ttaagagtct ctgggactgc tcttgtaaat gctccttgta    180

atttaaagga tattgacata acgaaatggt tgtgtaaaac agggagatta tatcttgata    240

aggttaagaa atttgaaata gttactattc tttcccatga cgtagaaaat caaaagatta    300

taacagaatg ggagtcactc cccagagagg ctttacccga acaatttgat tcataagaac    360

taattagtag cgctttccaa tggaggcgct tttttatttg ggtagttgca taccactaaa    420

gatgttcagg tgcacatgag cattggagga aaggaacgct ttagggggaa gggaaacctt    480

taaacagtct taatccccct tgattttatg ttctctgtaa actgcgtccg gtaaatctca    540

ggatagacaa tcggcggtta acggcttgag tgcgggggca gtttagaaag aatatgattg    600

gagggattca tagatgcatc accatcacca tcatatgagc gccaagtcca gaaccatagg    660

gattattgga gctcctttct caaagggaca gccacgagga ggggtggaag aaggccctac    720

agtattgaga aaggctggtc tgcttgagaa acttaaagaa caagagtgtg atgtgaagga    780

ttatggggac ctgccctttg ctgacatccc taatgacagt ccctttcaaa ttgtgaagaa    840

tccaaggtct gtgggaaaag caagcgagca gctggctggc aaggtggcac aagtcaagaa    900

gaacggaaga atcagcctgg tgctgggcgg agaccacagt ttggcaattg gaagcatctc    960

tggccatgcc agggtccacc ctgatcttgg agtcatctgg gtggatgctc acactgatat   1020

caacactcca ctgacaacca caagtggaaa cttgcatgga caacctgtat ctttcctcct   1080
```

```
gaaggaacta aaaggaaaga ttcccgatgt gccaggattc tcctgggtga ctccctgtat   1140

atctgccaag gatattgtgt atattggctt gagagacgtg gaccctgggg aacactacat   1200

tttgaaaact ctaggcatta aatacttttc aatgactgaa gtggacagac taggaattgg   1260

caaggtgatg gaagaaacac tcagctatct actaggaaga aagaaaaggc caattcatct   1320

aagttttgat gttgacggac tggacccatc tttcacacca gctactggca caccagtcgt   1380

gggaggtctg acatacagag aaggtctcta catcacagaa gaaatctaca aaacagggct   1440

actctcagga ttagatataa tggaagtgaa cccatccctg ggggaagacac cagaagaagt   1500

aactcgaaca gtgaacacag cagttgcaat aaccttggct tgtttcggac ttgctcggga   1560

gggtaatcac aagcctattg actaccttaa cccacctaag taaatgtgga aacatccgat   1620

ataaatctca tagttaatgg cataattaga aagctaatca ttttcttaag catagagtta   1680

tccttctaaa gacttgttct ttcagaaaaa tgtttttcca attagtataa actctacaaa   1740

ttccctcttg gtgtaaaatt caagatgtgg aaattctaac ttttttgaaa tttaaaagct   1800

tatattttct aacttggcaa aagacttatc cttagaaaga gaagtgtaca ttgatttcca   1860

attaaaaatt tgctggcatt aaaaataagc acacttacat aagcccccat acatagagtg   1920

ggactcttgg aatcaggaga caaagctacc acatgtggaa aggtactatg tgtccatgtc   1980

attcaaaaaa tgtgattcta ga                                            2002


<210>  2
<211>  990
<212>  DNA
<213>  Homo sapiens


<220>
<221>  CDS
<222>  (1)..(990)

<400>  2
atg cat cac cat cac cat cat atg agc gcc aag tcc aga acc ata ggg      48
Met His His His His His His Met Ser Ala Lys Ser Arg Thr Ile Gly
1               5                   10                  15

att att gga gct cct ttc tca aag gga cag cca cga gga ggg gtg gaa      96
Ile Ile Gly Ala Pro Phe Ser Lys Gly Gln Pro Arg Gly Gly Val Glu
            20                  25                  30

gaa ggc cct aca gta ttg aga aag gct ggt ctg ctt gag aaa ctt aaa     144
Glu Gly Pro Thr Val Leu Arg Lys Ala Gly Leu Leu Glu Lys Leu Lys
        35                  40                  45

gaa caa gag tgt gat gtg aag gat tat ggg gac ctg ccc ttt gct gac     192
Glu Gln Glu Cys Asp Val Lys Asp Tyr Gly Asp Leu Pro Phe Ala Asp
    50                  55                  60

atc cct aat gac agt ccc ttt caa att gtg aag aat cca agg tct gtg     240
```

```
Ile Pro Asn Asp Ser Pro Phe Gln Ile Val Lys Asn Pro Arg Ser Val
65                  70                  75                  80

gga aaa gca agc gag cag ctg gct ggc aag gtg gca caa gtc aag aag      288
Gly Lys Ala Ser Glu Gln Leu Ala Gly Lys Val Ala Gln Val Lys Lys
                85                  90                  95

aac gga aga atc agc ctg gtg ctg ggc gga gac cac agt ttg gca att      336
Asn Gly Arg Ile Ser Leu Val Leu Gly Gly Asp His Ser Leu Ala Ile
            100                 105                 110

gga agc atc tct ggc cat gcc agg gtc cac cct gat ctt gga gtc atc      384
Gly Ser Ile Ser Gly His Ala Arg Val His Pro Asp Leu Gly Val Ile
        115                 120                 125

tgg gtg gat gct cac act gat atc aac act cca ctg aca acc aca agt      432
Trp Val Asp Ala His Thr Asp Ile Asn Thr Pro Leu Thr Thr Thr Ser
    130                 135                 140

gga aac ttg cat gga caa cct gta tct ttc ctc ctg aag gaa cta aaa      480
Gly Asn Leu His Gly Gln Pro Val Ser Phe Leu Leu Lys Glu Leu Lys
145                 150                 155                 160

gga aag att ccc gat gtg cca gga ttc tcc tgg gtg act ccc tgt ata      528

Gly Lys Ile Pro Asp Val Pro Gly Phe Ser Trp Val Thr Pro Cys Ile
                165                 170                 175

tct gcc aag gat att gtg tat att ggc ttg aga gac gtg gac cct ggg      576
Ser Ala Lys Asp Ile Val Tyr Ile Gly Leu Arg Asp Val Asp Pro Gly
            180                 185                 190

gaa cac tac att ttg aaa act cta ggc att aaa tac ttt tca atg act      624
Glu His Tyr Ile Leu Lys Thr Leu Gly Ile Lys Tyr Phe Ser Met Thr
        195                 200                 205

gaa gtg gac aga cta gga att ggc aag gtg atg gaa gaa aca ctc agc      672
Glu Val Asp Arg Leu Gly Ile Gly Lys Val Met Glu Glu Thr Leu Ser
    210                 215                 220

tat cta cta gga aga aag aaa agg cca att cat cta agt ttt gat gtt      720
Tyr Leu Leu Gly Arg Lys Lys Arg Pro Ile His Leu Ser Phe Asp Val
225                 230                 235                 240

gac gga ctg gac cca tct ttc aca cca gct act ggc aca cca gtc gtg      768
Asp Gly Leu Asp Pro Ser Phe Thr Pro Ala Thr Gly Thr Pro Val Val
                245                 250                 255

gga ggt ctg aca tac aga gaa ggt ctc tac atc aca gaa gaa atc tac      816
Gly Gly Leu Thr Tyr Arg Glu Gly Leu Tyr Ile Thr Glu Glu Ile Tyr
            260                 265                 270

aaa aca ggg cta ctc tca gga tta gat ata atg gaa gtg aac cca tcc      864
Lys Thr Gly Leu Leu Ser Gly Leu Asp Ile Met Glu Val Asn Pro Ser
        275                 280                 285

ctg ggg aag aca cca gaa gaa gta act cga aca gtg aac aca gca gtt      912
Leu Gly Lys Thr Pro Glu Glu Val Thr Arg Thr Val Asn Thr Ala Val
    290                 295                 300

gca ata acc ttg gct tgt ttc gga ctt gct cgg gag ggt aat cac aag      960
```

```
Ala Ile Thr Leu Ala Cys Phe Gly Leu Ala Arg Glu Gly Asn His Lys
305                 310                 315                 320

cct att gac tac ctt aac cca cct aag taa                                  990
Pro Ile Asp Tyr Leu Asn Pro Pro Lys
                325
```

<210> 3
<211> 329
<212> PRT
<213> Homo sapiens

<400> 3

```
Met His His His His His His Met Ser Ala Lys Ser Arg Thr Ile Gly
1               5                   10                  15

Ile Ile Gly Ala Pro Phe Ser Lys Gly Gln Pro Arg Gly Gly Val Glu
            20                  25                  30

Glu Gly Pro Thr Val Leu Arg Lys Ala Gly Leu Leu Glu Lys Leu Lys
        35                  40                  45

Glu Gln Glu Cys Asp Val Lys Asp Tyr Gly Asp Leu Pro Phe Ala Asp
    50                  55                  60

Ile Pro Asn Asp Ser Pro Phe Gln Ile Val Lys Asn Pro Arg Ser Val
65                  70                  75                  80

Gly Lys Ala Ser Glu Gln Leu Ala Gly Lys Val Ala Gln Val Lys Lys
                85                  90                  95

Asn Gly Arg Ile Ser Leu Val Leu Gly Gly Asp His Ser Leu Ala Ile
            100                 105                 110

Gly Ser Ile Ser Gly His Ala Arg Val His Pro Asp Leu Gly Val Ile
        115                 120                 125

Trp Val Asp Ala His Thr Asp Ile Asn Thr Pro Leu Thr Thr Thr Ser
    130                 135                 140

Gly Asn Leu His Gly Gln Pro Val Ser Phe Leu Leu Lys Glu Leu Lys
145                 150                 155                 160

Gly Lys Ile Pro Asp Val Pro Gly Phe Ser Trp Val Thr Pro Cys Ile
                165                 170                 175

Ser Ala Lys Asp Ile Val Tyr Ile Gly Leu Arg Asp Val Asp Pro Gly
            180                 185                 190
```

```
Glu His Tyr Ile Leu Lys Thr Leu Gly Ile Lys Tyr Phe Ser Met Thr
        195                 200                 205

Glu Val Asp Arg Leu Gly Ile Gly Lys Val Met Glu Glu Thr Leu Ser
    210                 215                 220

Tyr Leu Leu Gly Arg Lys Lys Arg Pro Ile His Leu Ser Phe Asp Val
225                 230                 235                 240

Asp Gly Leu Asp Pro Ser Phe Thr Pro Ala Thr Gly Thr Pro Val Val
                245                 250                 255

Gly Gly Leu Thr Tyr Arg Glu Gly Leu Tyr Ile Thr Glu Glu Ile Tyr
            260                 265                 270

Lys Thr Gly Leu Leu Ser Gly Leu Asp Ile Met Glu Val Asn Pro Ser
        275                 280                 285

Leu Gly Lys Thr Pro Glu Glu Val Thr Arg Thr Val Asn Thr Ala Val
    290                 295                 300

Ala Ile Thr Leu Ala Cys Phe Gly Leu Ala Arg Glu Gly Asn His Lys
305                 310                 315                 320

Pro Ile Asp Tyr Leu Asn Pro Pro Lys
                325


<210>  4
<211>  7
<212>  PRT
<213>  Artificial

<220>
<223>  His Tag


<220>
<221>  MISC_FEATURE
<222>  (2)..(7)

<223>  6x HIS TAG

<400>  4

Met His His His His His His
1               5


<210>  5
<211>  33
```

<212>  DNA
<213>  Artificial

<220>
<223>  primer

<220>
<221>  misc_feature
<223>  PCR primer

<400>  5
ccaaaccata tgagcgccaa gtccagaacc ata    33

<210>  6
<211>  39
<212>  DNA
<213>  Artificial

<220>
<223>  primer

<220>
<221>  misc_feature
<223>  PCR primer

<400>  6
ccaaactcta gaatcacatt ttttgaatga catggacac    39

<210>  7
<211>  24
<212>  DNA
<213>  Artificial

<220>
<223>  primer

<220>
<221>  misc_feature
<223>  sequencing primer

<400>  7
ctctggccat gccagggtcc accc    24

<210>  8
<211>  969
<212>  DNA
<213>  Homo sapiens

<220>
<221>  CDS
<222>  (1)..(969)

<400>  8
atg agc gcc aag tcc aga acc ata ggg att att gga gct cct ttc tca    48

```
Met Ser Ala Lys Ser Arg Thr Ile Gly Ile Ile Gly Ala Pro Phe Ser
1               5                   10                  15

aag gga cag cca cga gga ggg gtg gaa gaa ggc cct aca gta ttg aga      96
Lys Gly Gln Pro Arg Gly Gly Val Glu Glu Gly Pro Thr Val Leu Arg
            20                  25                  30

aag gct ggt ctg ctt gag aaa ctt aaa gaa caa gag tgt gat gtg aag     144
Lys Ala Gly Leu Leu Glu Lys Leu Lys Glu Gln Glu Cys Asp Val Lys
        35                  40                  45

gat tat ggg gac ctg ccc ttt gct gac atc cct aat gac agt ccc ttt     192
Asp Tyr Gly Asp Leu Pro Phe Ala Asp Ile Pro Asn Asp Ser Pro Phe
    50                  55                  60

caa att gtg aag aat cca agg tct gtg gga aaa gca agc gag cag ctg     240
Gln Ile Val Lys Asn Pro Arg Ser Val Gly Lys Ala Ser Glu Gln Leu
65                  70                  75                  80

gct ggc aag gtg gca caa gtc aag aag aac gga aga atc agc ctg gtg     288
Ala Gly Lys Val Ala Gln Val Lys Lys Asn Gly Arg Ile Ser Leu Val
                85                  90                  95

ctg ggc gga gac cac agt ttg gca att gga agc atc tct ggc cat gcc     336
Leu Gly Gly Asp His Ser Leu Ala Ile Gly Ser Ile Ser Gly His Ala
            100                 105                 110

agg gtc cac cct gat ctt gga gtc atc tgg gtg gat gct cac act gat     384
Arg Val His Pro Asp Leu Gly Val Ile Trp Val Asp Ala His Thr Asp
        115                 120                 125

atc aac act cca ctg aca acc aca agt gga aac ttg cat gga caa cct     432
Ile Asn Thr Pro Leu Thr Thr Thr Ser Gly Asn Leu His Gly Gln Pro
    130                 135                 140

gta tct ttc ctc ctg aag gaa cta aaa gga aag att ccc gat gtg cca     480
Val Ser Phe Leu Leu Lys Glu Leu Lys Gly Lys Ile Pro Asp Val Pro
145                 150                 155                 160

gga ttc tcc tgg gtg act ccc tgt ata tct gcc aag gat att gtg tat     528
Gly Phe Ser Trp Val Thr Pro Cys Ile Ser Ala Lys Asp Ile Val Tyr
                165                 170                 175

att ggc ttg aga gac gtg gac cct ggg gaa cac tac att ttg aaa act     576
Ile Gly Leu Arg Asp Val Asp Pro Gly Glu His Tyr Ile Leu Lys Thr
            180                 185                 190

cta ggc att aaa tac ttt tca atg act gaa gtg gac aga cta gga att     624
Leu Gly Ile Lys Tyr Phe Ser Met Thr Glu Val Asp Arg Leu Gly Ile
        195                 200                 205

ggc aag gtg atg gaa gaa aca ctc agc tat cta cta gga aga aag aaa     672
Gly Lys Val Met Glu Glu Thr Leu Ser Tyr Leu Leu Gly Arg Lys Lys
    210                 215                 220

agg cca att cat cta agt ttt gat gtt gac gga ctg gac cca tct ttc     720
Arg Pro Ile His Leu Ser Phe Asp Val Asp Gly Leu Asp Pro Ser Phe
225                 230                 235                 240

aca cca gct act ggc aca cca gtc gtg gga ggt ctg aca tac aga gaa     768
Thr Pro Ala Thr Gly Thr Pro Val Val Gly Gly Leu Thr Tyr Arg Glu
```

```
                245                 250                 255

ggt ctc tac atc aca gaa gaa atc tac aaa aca ggg cta ctc tca gga       816
Gly Leu Tyr Ile Thr Glu Glu Ile Tyr Lys Thr Gly Leu Leu Ser Gly
            260                 265                 270

tta gat ata atg gaa gtg aac cca tcc ctg ggg aag aca cca gaa gaa       864
Leu Asp Ile Met Glu Val Asn Pro Ser Leu Gly Lys Thr Pro Glu Glu
        275                 280                 285

gta act cga aca gtg aac aca gca gtt gca ata acc ttg gct tgt ttc       912
Val Thr Arg Thr Val Asn Thr Ala Val Ala Ile Thr Leu Ala Cys Phe
    290                 295                 300

gga ctt gct cgg gag ggt aat cac aag cct att gac tac ctt aac cca       960

Gly Leu Ala Arg Glu Gly Asn His Lys Pro Ile Asp Tyr Leu Asn Pro
305                 310                 315                 320

cct aag taa                                                           969
Pro Lys



<210>  9
<211>  322
<212>  PRT
<213>  Homo sapiens

<400>  9

Met Ser Ala Lys Ser Arg Thr Ile Gly Ile Ile Gly Ala Pro Phe Ser
1               5                   10                  15


Lys Gly Gln Pro Arg Gly Gly Val Glu Glu Gly Pro Thr Val Leu Arg
            20                  25                  30


Lys Ala Gly Leu Leu Glu Lys Leu Lys Glu Gln Glu Cys Asp Val Lys
        35                  40                  45


Asp Tyr Gly Asp Leu Pro Phe Ala Asp Ile Pro Asn Asp Ser Pro Phe
    50                  55                  60


Gln Ile Val Lys Asn Pro Arg Ser Val Gly Lys Ala Ser Glu Gln Leu
65                  70                  75                  80


Ala Gly Lys Val Ala Gln Val Lys Lys Asn Gly Arg Ile Ser Leu Val
                85                  90                  95


Leu Gly Gly Asp His Ser Leu Ala Ile Gly Ser Ile Ser Gly His Ala
            100                 105                 110


Arg Val His Pro Asp Leu Gly Val Ile Trp Val Asp Ala His Thr Asp
        115                 120                 125
```

```
Ile Asn Thr Pro Leu Thr Thr Thr Ser Gly Asn Leu His Gly Gln Pro
    130                 135                 140

Val Ser Phe Leu Leu Lys Glu Leu Lys Gly Lys Ile Pro Asp Val Pro
145                 150                 155                 160

Gly Phe Ser Trp Val Thr Pro Cys Ile Ser Ala Lys Asp Ile Val Tyr
                165                 170                 175

Ile Gly Leu Arg Asp Val Asp Pro Gly Glu His Tyr Ile Leu Lys Thr
            180                 185                 190

Leu Gly Ile Lys Tyr Phe Ser Met Thr Glu Val Asp Arg Leu Gly Ile
        195                 200                 205

Gly Lys Val Met Glu Glu Thr Leu Ser Tyr Leu Leu Gly Arg Lys Lys
    210                 215                 220

Arg Pro Ile His Leu Ser Phe Asp Val Asp Gly Leu Asp Pro Ser Phe
225                 230                 235                 240

Thr Pro Ala Thr Gly Thr Pro Val Val Gly Gly Leu Thr Tyr Arg Glu
                245                 250                 255

Gly Leu Tyr Ile Thr Glu Glu Ile Tyr Lys Thr Gly Leu Leu Ser Gly
            260                 265                 270

Leu Asp Ile Met Glu Val Asn Pro Ser Leu Gly Lys Thr Pro Glu Glu
        275                 280                 285

Val Thr Arg Thr Val Asn Thr Ala Val Ala Ile Thr Leu Ala Cys Phe
    290                 295                 300

Gly Leu Ala Arg Glu Gly Asn His Lys Pro Ile Asp Tyr Leu Asn Pro
305                 310                 315                 320

Pro Lys
```